# EUROPEAN PATENT APPLICATION

(11) **EP 4 614 134 A1**
(43) Date of publication of application: **10.09.2025**
(21) Application number: 23884594.5
(22) Date of filing: 17.10.2023
(51) Int. Cl.: G01N 21/3504, G01N 33/00, G01L 11/00, G01N 21/01

(54) **GAS DETECTION DEVICE**

(30) Priority: 03.11.2022 CN 202211373708; 03.11.2022 CN 202211372374
(71) Applicant: Hangzhou Sanhua Research Institute Co., Ltd., Hangzhou, Zhejiang 310018 (CN)
(72) Inventor: WAN, Xia, Hangzhou, Zhejiang 310018 (CN); ZHANG, Jiajun, Hangzhou, Zhejiang 310018 (CN); LI, Linlong, Hangzhou, Zhejiang 310018 (CN); ZHANG, Yuxiang, Hangzhou, Zhejiang 310018 (CN); WANG, Cancan, Hangzhou, Zhejiang 310018 (CN); HUANG, Longzhong, Hangzhou, Zhejiang 310018 (CN)
(74) Representative: Ipside
(86) International application number: PCT/CN2023/124914
(87) International publication number: WO 2024/093662

(57) **Abstract**

This application provides a gas detection device, which includes a shell, a detection unit, and a circuit board. The detection unit is electrically connected to the circuit board. The detection unit includes a detection casing and has an air chamber. The detection casing is located on the periphery of the air chamber and has a mating hole in communication with the air chamber. The shell has a ventilation hole. At least part of the detection unit is located inside the shell, and at least part of the circuit board is located inside the shell. The ventilation hole is in communication with the mating hole and also in communication with the outside of the gas detection device. The shell is formed as a single integral structure. This simplifies the manufacturing and installation process.

## Description

### CROSS-REFERENCE TO RELATED APPLICATION

This application claims priority to Chinese Patent Application Nos. 202211373708.2 and 202211372374.7, both filed on November 3, 2022 and titled "Gas Detection Device". The contents of all these patent applications are incorporated herein by reference in their entireties.

### TECHNICAL FIELD

This application relates to the field of measurement technology and in particular, to a gas detection device.

### BACKGROUND

A gas detection device based on optical detection principles usually includes a shell, a detection module, and a circuit board. The detection module and the circuit board are located within the inner cavity of the shell, which protects the detection module and the circuit board.

In the prior art, the shell adopts a split structure, typically formed by assembling an upper casing and a lower casing, resulting in a complex structure with multiple components and high manufacturing costs. Therefore, there is a need to improve the structure of gas detection devices.

### SUMMARY

This application provides a gas detection device with simplified manufacturing and installation processes.

One aspect of the application is directed to a gas detection device. The gas detection device includes a detection unit, which includes a detection casing. The detection unit has an air chamber, with at least a portion of the detection casing is positioned on a peripheral of the air chamber. The detection casing has a mating hole that is in communication with the air chamber. The gas detection device further includes a circuit board, which is electrically connected to the detection unit. The gas detection device also includes a shell, within which at least a portion of the detection unit is located, and at least a portion of the circuit board is located. The shell has a ventilation hole that is in communication with the mating hole and an external environment of the gas detection device. The shell is formed as a single integral structure.

The disclosed gas detection device of this application has a shell formed as a single integral structure, thus simplifying the manufacturing and installation processes.

### BRIEF DESCRIPTION OF DRAWINGS

FIG. 1 is a perspective view of an exemplary gas detection device consistent with an embodiment of this application;
FIG. 2 is an exploded view of the exemplary gas detection device consistent with the embodiment of this application;
FIG. 3 is a top view of the exemplary gas detection device consistent with the embodiment of this application;
FIG. 4 is a sectional view along the A-A direction of the gas detection device shown in FIG. 3;
FIG. 5 is a perspective view of the exemplary shell of a gas detection device consistent with the embodiment of this application;
FIG. 6 is a top view of the exemplary shell of a gas detection device consistent with the embodiment of this application;
FIG. 7 is a sectional view along the B-B direction of the shell shown in FIG. 6;
FIG. 8 is a perspective view of the exemplary detection unit consistent with the embodiment of this application;
FIG. 9 is a perspective view of the exemplary base consistent with the embodiment of this application;
FIG. 10 is an exploded view of the exemplary detection unit consistent with the embodiment of this application;
FIG. 11 is a projection view of the exemplary detection unit consistent with the embodiment of this application;
FIG. 12 is a projection view of the exemplary shell consistent with the embodiment of this application.
FIG. 13 is a perspective view of another exemplary gas detection device consistent with another embodiment of this application;
FIG. 14 is an exploded view of the exemplary gas detection device consistent with the another embodiment of this application;
FIG. 15 is a sectional view of the exemplary gas detection device consistent with the another embodiment of this application;
FIG. 16 is an exploded view of the exemplary circuit board assembly consistent with the another embodiment of this application;
FIG. 17 is a perspective view of an exemplary detection unit consistent with the another embodiment of this application;
FIG. 18 is a perspective view of the exemplary detection unit consistent with the another embodiment of this application;
FIG. 19 is a partial sectional view of the exemplary detection unit consistent with the another embodiment of this application;
FIG. 20 is a partial sectional view of the exemplary detection unit consistent with the another embodiment of this application;
FIG. 21 is an exploded view of the exemplary detection unit consistent with the another embodiment of this application;
FIG. 22 is a perspective view of an exemplary shell consistent with the another embodiment of this application;
FIG. 23 is a sectional view of the exemplary shell consistent with the another embodiment of this application;
FIG. 24 is a projection view of the exemplary detection unit consistent with the another embodiment of this application; and
FIG. 25 is a projection view of the exemplary shell consistent with the another embodiment of this application.

### DETAILED DESCRIPTION

The following will provide a detailed explanation of the exemplary specific embodiments of this application in conjunction with the accompanying drawings. If there are multiple specific embodiments, the features of these embodiments can be combined with each other when there is no conflict. When the description refers to the drawings, unless otherwise stated, the same numbers in different drawings represent the same or similar elements. The content described in the following exemplary specific embodiments does not represent all implementations consistent with this application; on the contrary, they are only examples of devices, products and/or methods as claimed.

The terms used in this application are for the purpose of describing specific embodiments only and are not intended to limit the scope of protection of this application. The singular forms "a", "the" or "said" used in the specification and claims of this application are also intended to include plural forms, unless the context clearly indicates otherwise.

It should be understood that the use of terms such as "first", "second" and similar words in the specification and claims of this application does not indicate any order, quantity or importance, but is only used to distinguish the naming of features. Similarly, words like "one" or "a" do not indicate a quantity limitation, but indicate the existence of at least one. Unless otherwise indicated, similar words such as "front", "back", "up", "down" appearing in this application are only for convenience of description and are not limited to a particular position or spatial orientation. Words like "include" or "contain" are open-ended expressions, meaning that the elements appearing before "include" or "contain" cover the elements and their equivalents appearing after "include" or "contain", which does not exclude that the elements appearing before "include" or "contain" may also include other elements. If "several" appears in this application, it means two or more.

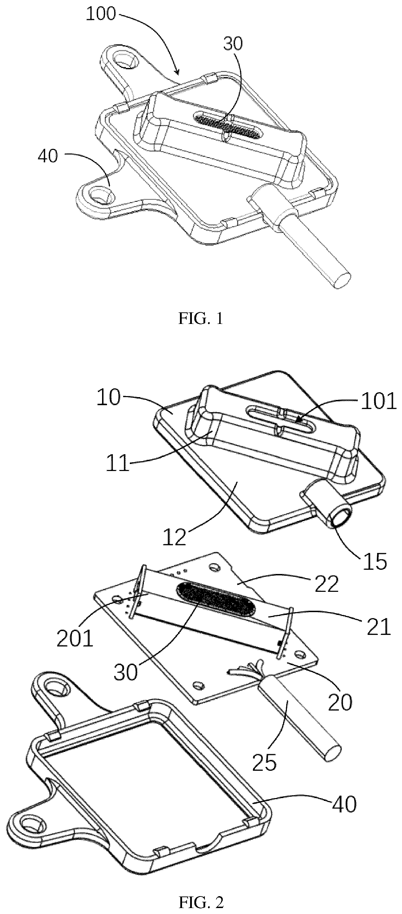

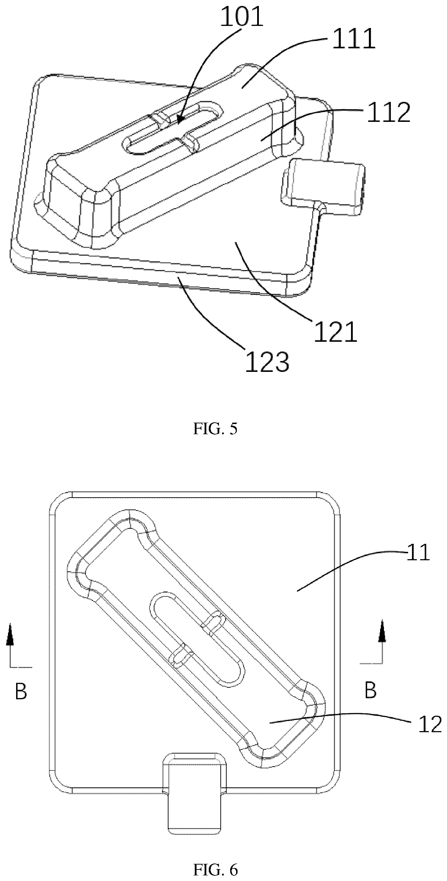

As shown in FIGS. 1 to 12, a gas detection device 100 consistent with an embodiment of this application includes: a shell 10 and a circuit board assembly 20. The circuit board assembly 20 includes a detection unit 21 and a circuit board 22. In some embodiments, the shell 10 has an inner cavity 200, so that at least part of the circuit board assembly 20 is located in the inner cavity 200. In some embodiments, the gas detection device 100 can be used to detect the concentration of gaseous refrigerant, so that when refrigerant leaks in an air conditioning system, it can be detected and fed back to the control system of the air conditioner in a timely manner, thereby reducing the safety hazards caused by refrigerant leakage. It is contemplated, in other embodiments, the gas detection device 100 can also be applied in other environments to detect other gases, such as methane, ethane, carbon dioxide and other gases. The scope of the present invention is not limited to the aforementioned examples.

The detection unit 21 is electrically connected to the circuit board 22, and the detection unit 21 is installed on the circuit board 22. The detection unit 21 is used to detect the concentration of gaseous refrigerants (such as environmentally friendly refrigerants like R32, R454B, etc.). The detection unit 21 described in the embodiments of this application uses optical detection principles. In some embodiments, the detection unit 21 can use infrared light detection principles. In other embodiments, depending on its working principle, the detection unit 21 can also be a semiconductor type, a thermal conductivity type, an electrochemical type, a catalytic combustion type, an ultrasonic type, etc.

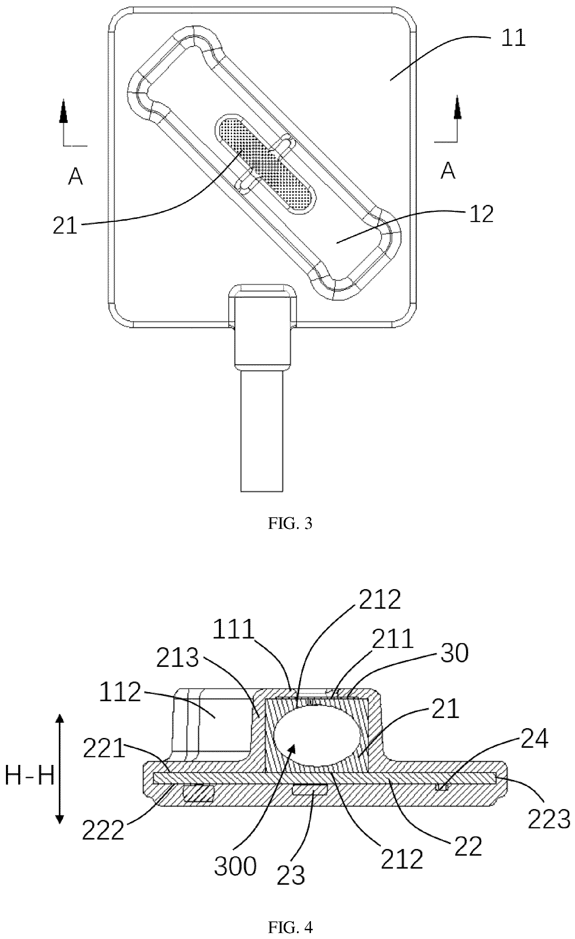

As shown in FIG. 4, the circuit board 22 also includes a processor chip 23 and several electronic components 24. The circuit board 22 has a first side 221 and a second side 222 on opposite sides in its thickness direction. The circuit board 22 has several conductive paths (not shown), at least part of which are electrically connected to the processor chip 23, and at least part of which are electrically connected to the electronic components 24.

The detection unit 21 is installed on the first side 221 of the circuit board 22. Meanwhile the processor chip 23 and a plurality of electronic components 24 are all installed on the second side 222 of the circuit board 22. It is contemplated, in other embodiments, the detection unit 21, processor chip 23, and several electronic components 24 can all be installed on the same side of the circuit board 22. The processor chip 23 is used to process the signal of the refrigerant gas concentration detected by the detection unit 21, and transmit it to an external control board or process it by itself. The electronic components 24 include filtering elements such as capacitors, resistors, inductors, etc., thus achieving amplification, filtering, and other effects on the signal coming from the detection unit 21.

In some embodiments, at least part of the detection unit 21 is located inside the shell 10, and at least part of the circuit board 22 is located inside the shell 10. In some embodiments, at least part of the detection unit 21 is located in the inner cavity 200, and at least part of the circuit board 22 is located in the inner cavity 200. The shell 10 serves to protect the detection unit 21 and the circuit board 22. As shown in FIG. 2, the shell 10 has a ventilation hole 101, and the ventilation hole 101 faces the detection unit 21.

In the embodiment illustrated in this application, the shell 10 is formed as a single integral structure. Specifically, the shell 10 is integrally injection-molded with the circuit board assembly 20 embedded therein as an insert. That is, the shell is formed by melting plastic, placing the circuit board assembly 20 into the mold, injecting the melted plastic into the mold and cooling, which is convenient to manufacture and form, with low cost.

In some embodiments, at least part of the detection unit 21 is in contact with the shell 10. This reduces the gap between the detection unit 21 and the shell 10, which helps to reduce the overall volume of the gas detection device 100. As shown in FIGS. 2 and 4, the shell 10 includes a first casing part 11, and at least part of the detection unit 21 is located within the first casing part 11. In other words, the first casing part 11 has a first inner cavity 13, and at least part of the detection unit 21 is located in the first inner cavity 13. The first casing part 11 is provided with the ventilation hole 101. At least part of the first casing part 11 is in contact with the detection casing 201, and at least part of the ventilation hole 101 is aligned with the mating hole 202. By encasing the detection unit 21 with the first casing part 11 of the shell 10, the overall height of the gas detection device 100 is reduced, decreasing the volume of the gas detection device 100, and saving occupied space, which allows for wider application scenarios.

In some embodiments, at least part of the circuit board 22 is in contact with the shell 10. As shown in FIGS. 2 and 4, the shell 10 includes a second casing part 12. The first casing part 11 is connected with the second casing part 12. At least part of the circuit board 22 is located within the second casing part 12. In other words, the second casing part 12 has a second inner cavity 14, and the circuit board 22 is located in the second inner cavity 14. At least part of the second casing part 12 is in contact with the circuit board 22. The first inner cavity 13 is in communication with the second inner cavity 14, and together they can form the inner cavity 200.

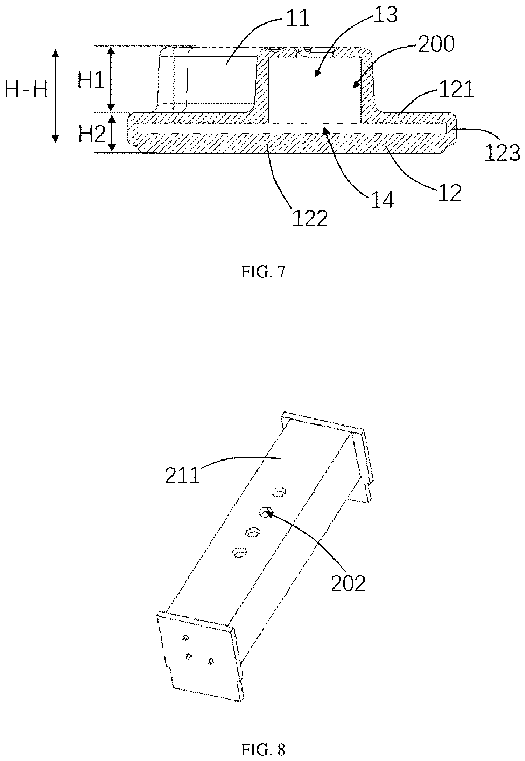

Specifically, as shown in FIGS. 2 and 4, the shell 10 includes a first casing part 11 and a second casing part 12, with the first casing part 11 integrally formed at the upper end of the second casing part 12. The circuit board 22 includes a first side 221 and a second side 222 located on different sides along its thickness direction H-H. Referring to FIG. 7, the first casing part 11 has a first height H1 along the thickness direction H-H, and the second casing part 12 has a second height H2 along the thickness direction H-H, where the first height H1 is greater than the second height H2. The thickness direction H-H of the circuit board 22 is aligned with the height direction of the gas detection device.

Referring to FIGS. 4, 5, and 7, the first casing part 11 includes a first wall part 111 and a first peripheral wall 112 extending perpendicularly outward from the first wall part 111. The first wall part 111 is provided with the ventilation hole 101. The detection unit 21 includes a detection casing 201. The detection casing 201 has an outer surface 211 and an inner surface 212, with the inner surface 212 being closer to the air chamber 300 relative to the outer surface 211. The mating hole 202 penetrates through the outer surface 211 and the inner surface 212. The outer surface 211 includes a first surface 2011, a second surface 2012, a third surface 2013, and a fourth surface 2014, which are connected in sequence. Along the height direction of the gas detection device 100, the first surface 2011 and the third surface 2013 are located on opposite sides of the detection casing 201, with the first surface 2011 being farther from the circuit board 22 relative to the third surface 2013. Along a direction perpendicular to the height direction of the gas detection device 100, the second surface 2012 and the fourth surface 2014 are located on opposite sides of the detection casing 201. The mating hole 202 penetrates through the first surface 2011. At least a portion of the first wall part 111 is in contact with the first surface 2011. At least a portion of the first peripheral wall 112 is in contact with at least one of the second surface 2012 and the fourth surface 2014. As shown in FIG. 4, the first surface 2011, the second surface 2012, and the fourth surface 2014 are all in contact with the first casing part 11, with the first casing part 11 tightly enclosing the detection casing 201. In some embodiments, the first casing part 11 tightly encloses the detection unit 21, with the detection unit 21 fitting snugly with the first casing part 11. Specifically, at least a portion of the outer surface of the detection unit 21 fits snugly with the first casing part 11.

Referring to FIGS. 4 and 7, the second casing part 12 includes a second wall part 121, a third wall part 122, and a second peripheral wall 123 which connects the second wall part 121 and the third wall part 122. The second wall part 121 and the third wall part 122 are located on different sides along the thickness direction H-H of the circuit board 22. The inner surface of the second wall part 121 is in contact with the first side 221 of the circuit board 22, and the inner surface of the third wall part 122 is in contact with the second side 222 of the circuit board 22. The circuit board 22 also includes a second peripheral surface 223, which is in contact with the inner surface of the second peripheral wall 123. The first peripheral wall 112 of the first casing part 11 is connected with the second wall part 121 of the second casing part 12. In some embodiments, the inner surface of the second wall part 121 fits snugly with the first side 221 of the circuit board 22, and the inner surface of the third wall part 122 fits snugly with the second side 222 of the circuit board 22. The circuit board 22 also includes a second peripheral surface 223, which fits snugly with the inner surface of the second peripheral wall 123. The second casing part 12 tightly encloses the circuit board 22, with the second casing part 12 fitting snugly with the circuit board 22.

The detection casing 201 can be a slender tubular casing, with the cross-section of the detection casing 201 being rectangular, circular, or other shapes. In this embodiment of the application, a detection casing 201 with a rounded rectangular outer contour in cross-section is illustrated. In other embodiments, a non-opposite reflection-type air chamber can also be constructed inside the detection casing 201, which means that the light emitted from the light source can reach the detection probe part after several reflections. In the following embodiments of this application, the explanation will mainly focus on the example of a tubular air chamber constructed inside the detection casing 201.

Referring to FIG. 4, the detection unit 21 also has an air chamber 300. The detection casing 201 is disposed around the air chamber 300. As shown in FIG. 8, the detection casing 201 is provided with a mating hole 202. The mating hole 202 penetrates through the detection casing 201. The mating hole 202 is in communication with the ventilation hole 101 and the air chamber 300. Specifically, the mating hole 202 penetrates through the outer surface 211 and the inner surface 212 of the detection casing 201. The ventilation hole 101 is in communication with the mating hole 202 and the external environment of the gas detection device 100. In some embodiments, the ventilation hole 101 is in direct communication with the mating hole 202 while being not in communication with the inner cavity 200. That is, at least a portion of the ventilation hole 101 is aligned with the mating hole 202, so that gas entering through the ventilation hole 101 directly enters the mating hole 202, and then enters the air chamber 300 through the mating hole 202.

Referring to FIG. 8, the detection casing 201 has at least two mating holes 202, which are arranged in a single row or at least two rows.

In some embodiments, the gas detection device 100 includes a waterproof gas-permeable membrane 30, which is connected to the outer surface 211 of the detection casing 201, and the waterproof gas-permeable membrane 30 covers the mating hole 202. At least a part of the waterproof gas-permeable membrane 30 is sandwiched between the outer surface 211 and the first wall part 111, as shown in FIG. 4. The waterproof gas-permeable membrane 30 reduces the possibility of water, dust, and other impurities from outside of the gas detection device 100 entering the air chamber 300, thus providing the gas detection device 100 with better waterproof and dustproof performance.

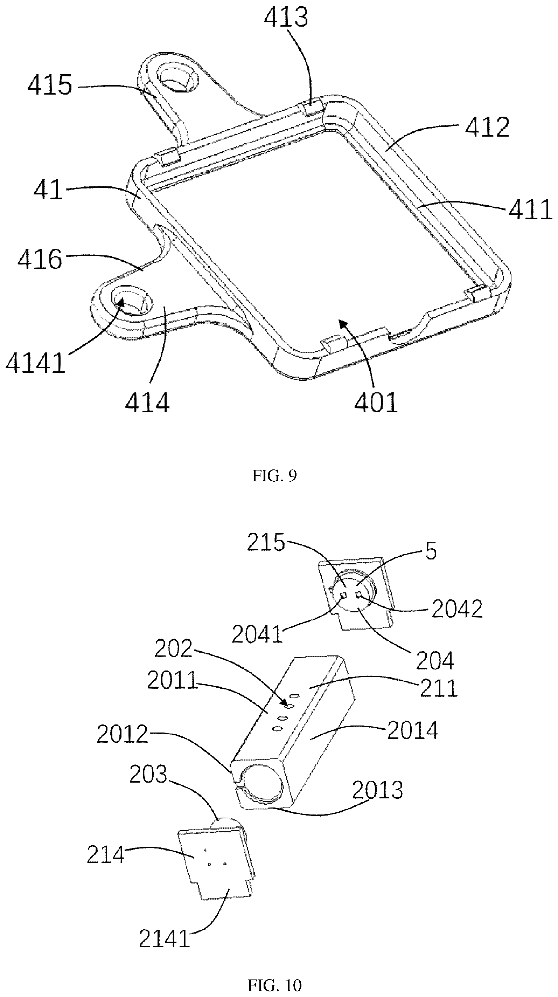

The gas detection device 100 also includes a base 40, which can be a plastic component with certain strength and hardness, with low material cost and can be manufactured through low-cost manufacturing methods such as injection molding. Referring to FIG. 9, the base 40 includes a supporting part 41, which can be a closed ring structure. The supporting part 41 has a through slot 401, and at least a part of the shell 10 is installed within the through slot 401. Specifically, at least a part of the shell 10 can be clamped and fixed in the through slot 401 of the supporting part 41, or it can be disposed in the through slot 401 of the supporting part 41 through adhesive bonding.

In some embodiments, the supporting part 41 includes a bottom wall 411, a third peripheral wall 412, and a boss 413. The through slot 401 extends through the bottom wall 411, the third peripheral wall 412 is located on the periphery of the through slot 401, and the boss 413 protrudes from the third peripheral wall 412 towards the through slot 401. Along the height direction of the gas detection device, the through slot 401 is partially located between the bottom wall 411 and the boss 413, and the height direction of the gas detection device is parallel to the thickness direction of the circuit board. Specifically, referring to FIG. 9, the supporting part 41 includes a ring-shaped bottom wall 411 and a third peripheral wall 412 extending vertically from the bottom wall 411, with the bottom wall 411 and the third peripheral wall 412 located on the periphery of the through slot 401. In the embodiment shown in this application, the supporting part 41 includes a boss 413 protruding from the third peripheral wall 412 towards the through slot 401, so that the boss 413 can clamp the shell 10. Specifically, the second casing part 12 of the shell 10 slides past the boss 413 and is installed in the through slot 401, with the boss 413 serving as a limiting function. The boss 413 has an overall triangular protrusion shape with a larger thickness at the bottom and a smaller thickness at the top, making it easy for the second casing part 12 of the shell 10 to slide down along the slanting surface of the boss 413 and finally be clamped and installed in the through slot 401. The bottom wall 411 provides axial limitation for the second casing part 12 of the shell 10, while the third peripheral wall 412 provides radial limitation for the second casing part 12 of the shell 10.

Continuing to refer to FIG. 9, the base 40 also includes a lug 414, which protrudes outward from the supporting part 41. The lug 414 has a mounting hole 4141 for assembly with other components. In the embodiment shown in this application, the lug 414 includes a first lug 415 and a second lug 416, with the first lug 415 and the second lug 416 located on two adjacent edges of the supporting part 41. It is contemplated, in other embodiments, the first lug 415 and the second lug 416 can also be arranged on two opposite edges of the supporting part 41.

Referring to FIG. 10, the detection unit 21 also includes a light source module 203 and a detection probe 204. The light source module 203 is located at one end of the detection casing 201 in the longitudinal direction, used to emit light, while the detection probe 204 is located at the other end of the detection casing 201 in the longitudinal direction, used to receive light. The light source module 203 can be chosen as an infrared light source, and correspondingly, the detection probe 204 is an infrared detection probe. The light source module 203 and the detection probe 204 are almost coaxially arranged, and the detection casing 201 is cylindrical, allowing the infrared light emitted by the light source module 203 to travel almost in a straight line to the detection probe 204. The light source module 203 and the detection probe 204 are electrically connected to the circuit board 22 respectively.

The principle of the above-mentioned detection unit 21 is explained as follows: different gases have their own distinct absorption spectra due to differences in their molecular structure, concentration, and energy distribution. When detecting target gases, the absorption of light at characteristic wavelengths by the target gas follows the Lambert-Beer law. Taking an infrared light source which acts as the light source module 203 as an example, when a beam of infrared light emitted by the light source module 203 passes through the air chamber 300 to reach the detection probe 204, the target gas absorbs infrared light at specific wavelengths. In other words, the target gas from the outside passes through structures such as the ventilation hole 101 of the shell 10, the waterproof gas-permeable membrane 30, and the mating hole 202 to enter the air chamber 300. The target gas that enters the air chamber 300 absorbs infrared light at specific wavelengths, so the detection probe 204 can calculate information such as the concentration of the target gas by detecting changes in light intensity.

The material of the detection casing 201 can be aluminum. In practice, in order to enhance light transmission inside the air chamber 300 and reduce light loss, the inner surface of the detection casing 201 can be polished and plated with metals such as gold, silver, aluminum, or chrome. It is contemplated that the material of the detection casing 201 can also be ABS plastic, with its inner surface gold-plated to enhance light emission and reflection effects. Additionally, the light source module 203 and detection probe 204 are arranged face-to-face. In order to ensure that the light emitted by the light source module 203 can travel as straight as possible to the detection probe 204, a reflector cup can be added near the light-emitting position of the light source module 203. The reflector cup can be trumpet-shaped, with one end narrowed and the other end widened. The reflector cup is nested inside the air chamber 300, with its outer periphery abutting or contacting the inner surface of the detection casing 201. The light-emitting element can be placed at the narrowed end of the reflector cup. As a type of reflector device, the reflector cup can utilize limited light energy to control the illumination distance and area of the main light spot of the light-emitting element through light reflection.

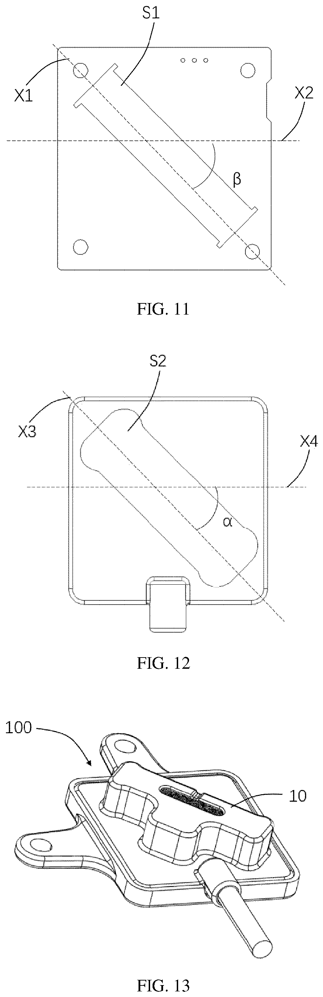

In order to improve the detection accuracy of the gas detection device 100, the cylindrical detection casing 201 needs to have a certain length. To achieve the certain length of the detection casing 201 in a confined space, refer to FIG. 11. In this application, a first line X1 and a second line X2 are defined on the first side 221 of the circuit board 22. Along the thickness direction of the circuit board 22, the detection casing 201 has a first projection S1 on the first side 221 of the circuit board 22. The first line X1 extends along the length direction of the first projection S1. The second line X2 extends along the width direction of the circuit board 22. A first angle β between the first line X1 and the second line X2 is an acute angle. This arrangement is beneficial for expanding the installation space of the detection unit 21, and correspondingly, for extending the distance between the light source module 203 and the detection probe 204. Thus, through a longer optical path, the gas absorption of infrared light can be more complete, which is conducive to improving the detection accuracy of the gas detection device 100.

Referring to FIG. 10, the detection unit 21 also includes a first adapter board 214 and a second adapter board 215. The first adapter board 214 has a first connecting part 2141, and the second adapter board 215 has a second connecting part 2151. The circuit board 22 is provided with corresponding connecting holes (not shown) for the first connecting part 2141 and the second connecting part 2151. The connecting part 2141 is at least partially located in the connecting hole. The pins of the light source module 203 are soldered to the first adapter board 214, and the pins of the detection probe 204 are soldered to the second adapter board 215. Both the first adapter board 214 and the second adapter board 215 are soldered to the circuit board 22.

Referring to FIG. 12, the first casing part 11 has a second projection S2 on the second casing part 12 along the thickness direction H-H. A third line X3 and a fourth line X4 are defined on the outer surface of the second wall part 121. The third line X3 extends along the length direction of the second projection S2. The fourth line X4 extends along the width direction of the second casing part 12. A second angle α between the third line X3 and the fourth line X4 is an acute angle. The second angle α is equal to the first angle β. In this way, the shape of the shell 10 is compatible with the structure of the circuit board assembly 20.

The light source module 203 is a MEMS-type blackbody light source or an incandescent light source, and the infrared light emitted by the light source module 203 has a peak wavelength range of 1µm to 16µm. Referring to FIG. 10, the detection probe 204 is a pyroelectric or thermopile type detection probe. The detection probe 204 includes independently arranged detection channel 2041 and reference channel 2042, both of which contain matching narrow-band filters and pyroelectric chips/thermopile chips. With this arrangement, since both the detection channel 2041 and reference channel 2042 of the detection probe 204 are affected by temperature, humidity, and cross-interference between different gases, the ratio or difference of their voltage output signals can be used to improve the accuracy of gas concentration detection by the detection channel 2041 when performing concentration calculations.

Referring to FIG. 2, the circuit board assembly 20 also includes a cable 25, which is electrically connected to the circuit board 22. The shell 10 also has a lead-out part 15 with a channel through which the cable 25 passes to connect electrically with the circuit board 22. The channel is in communication with the inner cavity 200.

This application encloses the detection unit 21 within the shell 10, which not only reduces the overall volume of the gas detection device but also allows gas to enter the air chamber 300 more quickly, improving the time response of the gas detection device 100 to gases. This enables rapid gas detection and quick alarm activation.

In some embodiments, the gas detection device 100 includes a pressure detection unit. The pressure detection unit is used to perform pressure compensation on the detection results of the gas detection unit, thereby improving the detection accuracy of the gas detection unit. The gas detection unit and the pressure detection unit each have their own shells with ventilation holes. The gas detection unit and the pressure detection unit each have their own air chambers, where the gas detection unit detects the gas in its air chamber, and the pressure detection unit detects the pressure of the gas in its air chamber. The gas detection unit includes a gas detection assembly 5, which includes the aforementioned light source module 203 and detection probe 204. The pressure detection unit includes a pressure detection assembly 6, which is used to detect gas pressure. The light source module 203, detection probe 204, and pressure detection assembly 6 are all electrically connected to the circuit board 22.

In some embodiments, the structure of the gas detection device is improved to allow the pressure detection unit to accurately compensate for the results of the gas detection unit, thereby improving detection accuracy.

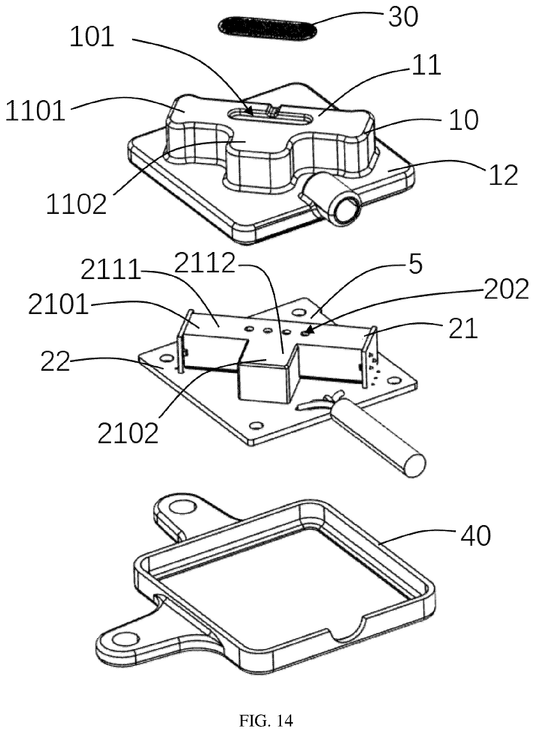

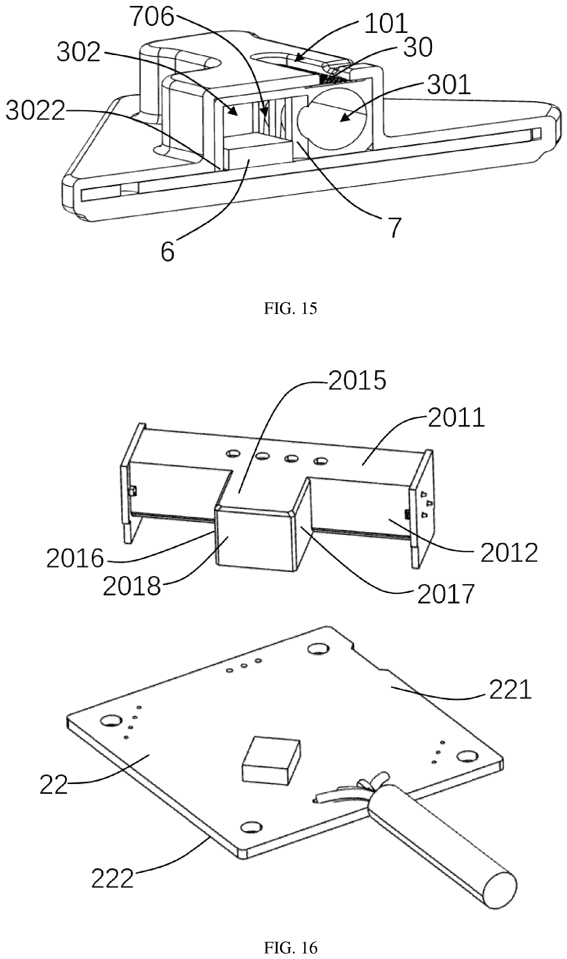

In some embodiments, as shown in FIGS. 13 to 23, the detection unit 21 includes a first detection unit 2101 and a second detection unit 2102, the air chamber 300 includes a first air chamber 301 and a second air chamber 302, and the detection casing 201 includes a first detection casing 2111 and a second detection casing 2112. The first detection unit 2101 includes a gas detection assembly 5 and the first detection casing 2111, the first detection unit 2101 has a first air chamber 301, the gas detection assembly 5 and the first detection casing 2111 are both arranged around the periphery of the first air chamber 301, the first detection casing 2111 is provided with the mating hole 202, and the mating hole 202 is in communication with the first air chamber 301. The second detection unit 2102 includes a pressure detection assembly 6 and the second detection casing 2112, the second detection unit 2102 has a second air chamber 302, the second detection casing 2112 is arranged around the periphery of the second air chamber 302, the pressure detection assembly 6 is located in the second air chamber 302; the second air chamber 302 is in communication with the first air chamber 301.

In the gas detection device of this application, the first detection unit 2101 includes the gas detection assembly 5 and the first detection casing 2111, which can perform gas detection; the second detection unit 2102 includes the pressure detection assembly 6 and the second detection casing 2112, which can perform pressure detection. The first detection unit 2101 has a first air chamber 301, the first detection casing 2111 is arranged around the periphery of the first air chamber 301, the first detection casing 2111 is provided with a mating hole 202, and the mating hole 202 is in communication with the first air chamber 301 and the external environment of the first detection unit 2101. The second detection unit 2102 has a second air chamber 302, the second air chamber 302 is in communication with the first air chamber 301, and the second air chamber 302 is in communication with the external environment of the gas detection device through the mating hole 202 set in the first detection casing 2112. In this way, the pressure in the second air chamber 302 tends to be the same as that in the first air chamber 301, which improves the accuracy of pressure compensation by the pressure detection assembly 6 for the gas detection assembly 5, thereby enhancing the detection precision of the gas detection device 100.

In some embodiments, the detection casing 201 has a through hole 706, the through hole 706 is in communication with the first air chamber 301 and the second air chamber 302, and the through hole 706 and the mating hole 202 are located on adjacent sides of the first air chamber 301.

Specifically, the first detection casing 2111 includes the aforementioned first surface 2011, second surface 2012, third surface 2013, and fourth surface 2014. The second detection casing 2112 includes a fifth surface 2015, sixth surface 2016, seventh surface 2017, and eighth surface 2018. The fifth surface 2015, sixth surface 2016, seventh surface 2017, and eighth surface 2018 are connected to each other. The fifth surface 2015 is in the same plane as the first surface 2011. Along the direction perpendicular to the height of the gas detection device, the sixth surface 2016 and seventh surface 2017 are located on opposite sides of the second air chamber 302. The eighth surface 2018 is disposed approximately perpendicular to the sixth surface 2016, and the eighth surface 2018 and the through hole 706 are located on opposite sides of the second air chamber 302.

In some embodiments, along the extending direction of the through hole 706, the first air chamber 301 and the second air chamber 302 are located on opposite sides of the through hole 706.

In some embodiments, the gas detection device includes a partition plate 7, which is connected to at least one of the first detection casing 2111 and the second detection casing 2112. The partition plate 7 is located between the first air chamber 301 and the second air chamber 302, and the partition plate 7 is provided with the through hole 706. The partition plate 7 includes a first face 701, which is exposed to the second air chamber 302. The partition plate 7 has a groove 703 that is recessed from the first face 701 into the partition plate 7, and the through hole 706 is in communication with the groove 703. The partition plate 7 includes a through-hole part 7061 and a groove part 7031. The through-hole part 7061 includes the through hole 706 and a corresponding through-hole wall 7062. The groove part 7031 includes the groove 703 and a corresponding groove wall 7032. Defining a plane perpendicular to the extending direction of the through hole 706 as the projection plane, along the thickness direction of the partition plate 7, an orthographic projection of the through-hole part 7061 on the projection plane is within an outer contour of the orthographic projection of the groove part 7031 on the projection plane. In some embodiments, along a direction perpendicular to the extending direction of the through hole 706, the groove part 7031 extending through the partition plate 7.

In some embodiments, the gas detection assembly 5 includes a light source module 203 and a detection probe 204. The light source module 203 and the detection probe 204 are connected with the first detection casing 2111 respectively. Along a length direction of the first detection casing 2111, the light source module 203 and the detection probe 204 are located at opposite ends of the first detection casing, with at least part of the light source module 203 and at least part of the detection probe 204 located around the periphery of the first air chamber 301. Along the length direction of the first detection casing 2111, the second detection casing 2112 is closer to the detection probe 204 relative to the light source module 203, and closer to the light source module 203 relative to the detection probe 204.

In some embodiments, the second air chamber 302 has an opening 3021. Along the thickness direction of the circuit board 22, the mating hole 202 and the opening 3021 are located on opposite sides of the air chamber 300. The second detection casing 2112 has an opening part 3022, which includes the opening 3021. The circuit board 22 covers the opening 3021. In other words, the circuit board 22 is located on the periphery of the second air chamber 302, with at least a portion of the circuit board 22 exposed to the second air chamber 302. Along the thickness direction of the circuit board 22, the projection of the opening part 3022 on the circuit board 22 is within the outer contour of the circuit board 22. This arrangement allows the second detection casing 2112 to be fitted over the pressure detection assembly 6 after the installation of the pressure detection assembly 6 on the circuit board 22. After assembling the detection casing 201 and the circuit board 22, the pressure detection assembly 6 is located in the second air chamber 302. Along the thickness direction of the circuit board 22, at least a portion of the circuit board 22 and the mating hole 202 are located on opposite sides of the detection casing 201. Specifically, along the height direction of the gas detection device 100, the opening 3021 and the fifth surface 2015 are located on opposite sides of the second air chamber 302. The height direction of the gas detection device 100 is parallel to the thickness direction of the circuit board 22.

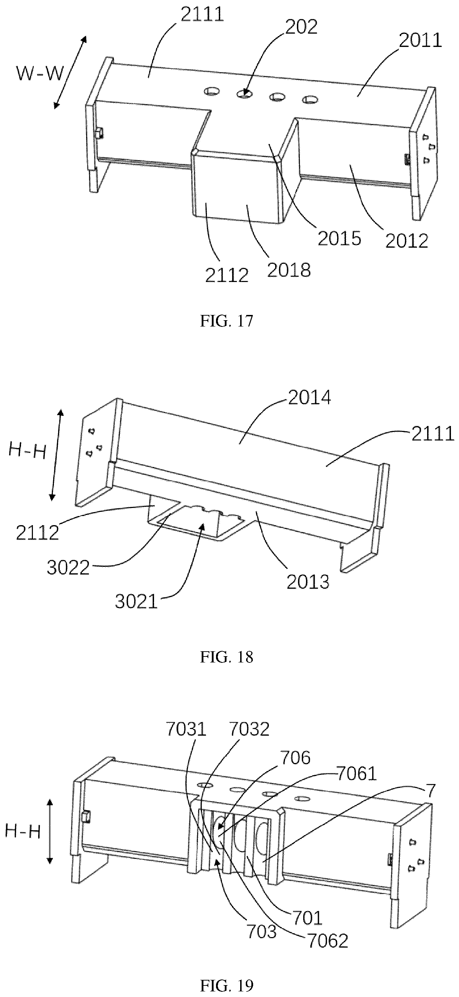

In some embodiments, the second detection casing 2112 is connected with the first detection casing 2111, and the detection casing 201 is formed as a single integral structure. The second detection casing 2112 is located on one side of the first detection casing 2111 along the width direction W-W, as shown in FIG. 17.

In some embodiments, the first casing part 11 includes a first sub-casing part 1101 and a second sub-casing part 1102, with the first sub-casing part 1101 is provided with the ventilation hole 101. The first detection unit 2101 is located within the first sub-casing part 1101, with at least a portion of the first sub-casing part 1101 fitting against the first detection unit 2101. The second detection unit 2102 is located within the second sub-casing part 1102, with at least a portion of the second sub-casing part 1102 fitting against the second detection unit 2102. Specifically, the first surface 2011 of the first detection casing 2111 and the fifth surface 2015 of the second detection casing 2112 both fit against the first wall part 111. The first peripheral wall 112 includes a first sub-peripheral wall 1121 and a second sub-peripheral wall 1122. The first sub-peripheral wall 1121 fits against the gas detection assembly 5, while the sixth surface 2016, seventh surface 2017, and eighth surface 2018 of the second detection casing 2112 all fit against the second sub-peripheral wall 1122.

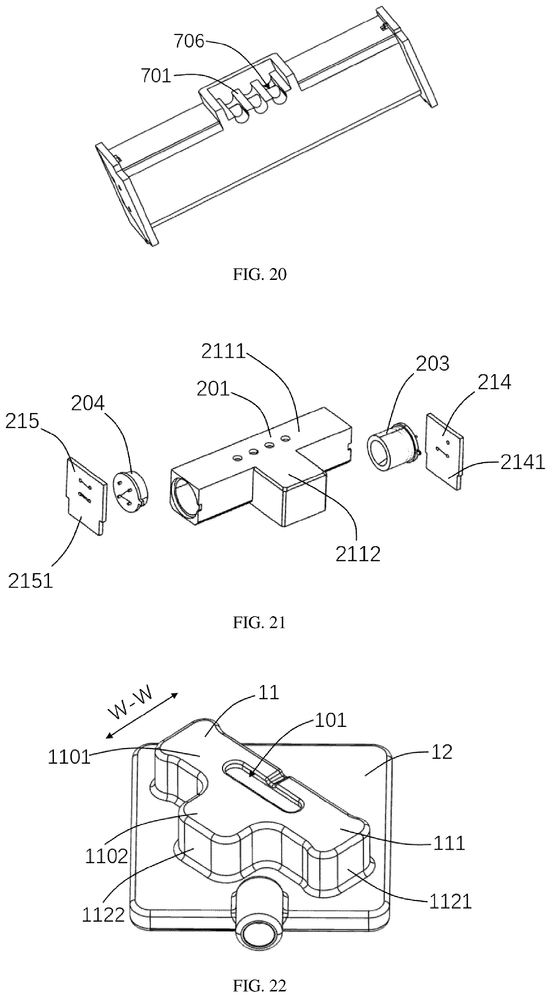

In some embodiments, both the first sub-casing part 1101 and the second sub-casing part 1102 are connected with the second casing part 12. Along the height direction of the gas detection device, the first sub-casing part 1101 and the second sub-casing part 1102 are located on the same side of the second casing part 12. The second sub-casing part 1102 is located on one side of the first sub-casing part 1101 along the width direction W-W, as shown in FIG. 22. The width direction of the first sub-casing part 1101 is aligned with the width direction of the first detection casing 2111.

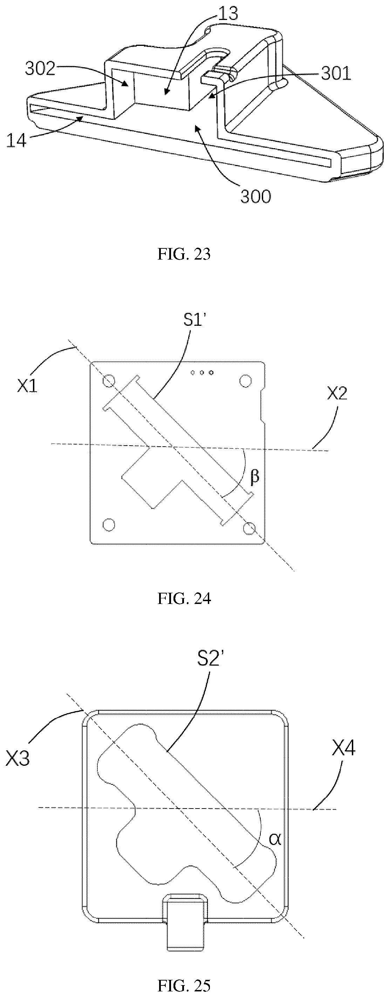

In some embodiments, as shown in FIG. 24, along the thickness direction of the circuit board 22, the first projection S1 of the detection casing 201 on the first side 221 of the circuit board 22 includes a first projection part S1', the first projection part S1' is the projection of the first detection casing 2111 on the first side 221 of the circuit board 22. A first line X1 extends along the length direction of the first projection S1'. Referring to FIG. 25, along the thickness direction H-H of the circuit board 22, the second projection S2 of the first casing part 11 on the second casing part 12 includes a second projection part S2', the second projection part S2' is the projection of the first sub-casing part 1101 on the second casing part 12. A third line X3 extends along the length direction of the second projection part S2'.

The above embodiments are only used to illustrate the present application and are not intended to limit the technical solutions described in this application. The understanding of this specification should be based on the technical personnel in the relevant field. Although this specification has described the present application in detail with reference to the above embodiments, those skilled in the art should understand that the technical personnel in the relevant field can still modify or equivalently replace the present application, and all technical solutions and improvements that do not deviate from the spirit and scope of the present application, which is defined by the claims.

## Claims

1. A gas detection device, comprising:
a detection unit (21), comprising a detection casing (201), wherein the detection unit (21) has an air chamber (300), the detection casing (201) is located at least partially on a periphery of the air chamber (300), and the detection casing (201) has a mating hole (202) in communication with the air chamber (300);
a circuit board (22) electrically connected to the detection unit (21); and
a shell (10), wherein at least part of the detection unit (21) is located inside the shell (10), at least part of the circuit board (22) is located inside the shell (10), the shell has a ventilation hole (101) in communication with the mating hole (202), and the ventilation hole (101)is in communication with an external environment of the gas detection device, wherein the shell (10)is formed as a single integral structure.

2. The gas detection device according to claim 1, wherein the shell (10) is an integrally injection-molded piece, the shell (10) comprises a first casing part (11), the detection unit (21) is at least partially located within the first casing part (11), and the first casing part (11) is provided with the ventilation hole (101); and
at least a portion of the first casing part (11) is in contact with the detection casing (201).

3. The gas detection device according to claim 2, wherein the first casing part (11) comprises a first wall part (111) and a first peripheral wall (112), the first peripheral wall (112) extends outward from the first wall part (111), and the first wall part (111) is provided with the ventilation hole (101);
the detection casing (201) has an outer surface, and the mating hole (202) penetrates the outer surface (211); and
at least a portion of the first wall part (111) is in contact with the outer surface (211).

4. The gas detection device according to claim 3, wherein the detection unit (21) comprises a waterproof gas-permeable membrane (30), the waterproof gas-permeable membrane (30) is connected to the outer surface (211), and the waterproof gas-permeable membrane (30) covers the mating hole (202), and at least a portion of the waterproof gas-permeable membrane (30) is sandwiched between the outer surface (211) and the first wall part (111).

5. The gas detection device according to claim 2, wherein the shell (10) further comprises a second casing part (12), the first casing part (11) is connected to the second casing part (12), the circuit board (22) is at least partially located within the second casing part (12), and the second casing part (12) is at least partially in contact with the circuit board (22), and the first casing part (11) and the second casing part (12) are integrally injection-molded;
the second casing part (12) comprises a second wall part (121), a third wall part (122) and a second peripheral wall (123), the second peripheral wall (123) connects the second wall part (121) and the third wall part (122), and along a thickness direction of the circuit board (22), the second wall part (121) and the third wall part (122) are located on different sides of the circuit board (22); and
the circuit board (22) comprises a first side (221), a second side (222), and a second peripheral surface (223), the second wall part (121) is in contact with the first side (221) of the circuit board (22), the third wall part (122) is in contact with the second side (222) of the circuit board (22), and the second peripheral surface (223) is in contact with the second peripheral wall (123).

6. The gas detection device according to claim 5, wherein a first line (X1) and a second line (X2) are defined on the first side (221) of the circuit board (22), along the thickness direction of the circuit board (22), the detection casing (201) has a first projection (S1) on the first side (221) of the circuit board (22), wherein the first line (X1) extends along a length direction of the first projection (S1), the second line (X2) extends along a width direction of the circuit board (22), and a first angle(β) between the first line (X1)and the second line (X2) is an acute angle.

7. The gas detection device according to claim 6, wherein along the thickness direction of the circuit board (22), the first casing part (11) has a second projection (S2) on the second casing part (12), a third line (X3) and a fourth line (X4) are defined on an outer surface of the second wall part (121), the third line (X3) extends along a length direction of the second projection (S2), and the fourth line (X4) extends along a width direction of the second casing part (12), a second angle (α) between the third line (X3) and the fourth line (X4) is an acute angle, and the second angle (α) is equal to the first angle (β).

8. The gas detection device according to claim 5, wherein the shell (10) is injection-molded with the detection unit (21) and the circuit board (22) as inserts, the first casing part (11) is snugly fitted with the detection unit (21), and the second casing part (12) is snugly fitted with the circuit board (22); and
along the thickness direction of the circuit board (22), the first casing part (11) has a first height (H1), the second casing part (12) has a second height (H2), and the first height (H1) is greater than the second height (H2).

9. The gas detection device according to claim 1, further comprising a base (40), wherein the base (40) comprises a supporting part (41), the supporting part (41) has a through slot (401), and at least a portion of the shell (10) is installed in the through slot (401); and
the gas detection device has a first state and a second state, in the first state, the shell (10) is fixedly connected or limitedly connected to the supporting part (41), and in the second state, the casing (10) is separated from the supporting part (41), wherein the gas detection device is configured to switch from the first state to the second state or from the second state to the first state.

10. The gas detection device according to claim 9, wherein the supporting part (41) comprises a bottom wall (411), a third peripheral wall (412), and a boss (413), wherein the through slot (401) extends through the bottom wall (411), the third peripheral wall (412) is located around a periphery of the through slot (401), and the boss (413) protrudes from the third peripheral wall (412) towards the through slot (401); and
along a height direction of the gas detection device, the through slot (401) is partially located between the bottom wall (401) and the boss (413), and the height direction of the gas detection device is parallel to a thickness direction of the circuit board.

11. The gas detection device according to claim 1, wherein the detection unit (21) comprises a first detection unit (2101) and a second detection unit (2102), the air chamber (300) comprises a first air chamber (301) and a second air chamber (302), and the detection casing (201) comprises a first detection casing (2111) and a second detection casing (2112);
the first detection unit (2101)comprises a gas detection assembly (5) and the first detection casing (2111), the first detection unit (2101) has the first air chamber (301), the gas detection assembly (5) and the first detection casing (2111) are both arranged around a periphery of the first air chamber (301), the first detection casing (2111) is provided with the mating hole (202), and the mating hole (202) is in communication with the first air chamber (301); and
the second detection unit (2102) comprises a pressure detection assembly (6) and the second detection casing (2112), the second detection unit (2102) has the second air chamber (302), the second detection casing (2112) is arranged around a periphery of the second air chamber (302), and the pressure detection assembly (6) is located in the second air chamber (302); and the second air chamber (302) is in communication with the first air chamber (301).

12. The gas detection device according to claim 11, wherein the detection casing (201) has a through hole (706), the through hole (706) communicates the first air chamber (301) and the second air chamber (302), and the through hole (706) and the mating hole (202) are located on adjacent sides of the first air chamber (301) respectively.

13. The gas detection device according to claim 12, wherein along an extending direction of the through hole (706), the first air chamber (301) and the second air chamber (302) are located on opposite sides of the through hole (706) respectively.

14. The gas detection device according to claim 13, wherein the gas detection device comprises a partition plate (7), the partition plate (7) is connected to at least one of the first detection casing (2111) and the second detection casing (2112), the partition plate (7) is located between the first air chamber (301) and the second air chamber (302), and the partition plate (7) is provided with the through hole (706);
the partition plate (7) comprises a first face (701), the first face (701) is exposed to the second air chamber (302), the partition plate (7) has a groove (703), the groove (703) is recessed from the first face (701) into the partition plate (7), and the through hole (706) is in communication with the groove (703); and
the partition plate (7) comprises a through-hole part (7061) and a groove part (7031), the through-hole part (7061) comprises the through hole (706) and a through-hole wall (7062) corresponding to the through hole (706), and the groove part (7031) comprises the groove (703) and a groove wall (7032) corresponding to the groove (703); a projection plane is defined as a plane perpendicular to the extending direction of the through hole (706), along a thickness direction of the partition plate (7), an orthographic projection of the groove part (7031) on the projection plane is defined as a first projection, an orthographic projection of the through-hole part (7061) on the projection plane is defined as a second projection, and the second projection is located within the outer contour of the first projection.

15. The gas detection device according to claim 14, wherein along a direction perpendicular to the extending direction of the through hole (706), the groove part (7031) extends through the partition plate (7).

16. The gas detection device according to claim 11, wherein the gas detection assembly (5) comprises a light source module (203) and a detection probe (204), and the light source module (203) and the detection probe (204) are connected to the first detection casing respectively (2111); along a length direction of the first detection casing (2111), the light source module (203) and the detection probe (204) are located at opposite ends of the first detection casing, and at least a portion of the light source module (203) and at least a portion of the detection probe (204) are both located at the periphery of the first air chamber (301); and
along the length direction of the first detection casing (2111), the second detection casing (2112) is closer to the detection probe (204) relative to the light source module (203), and the second detection casing (2112) is closer to the light source module (203) relative to the detection probe (204).

17. The gas detection device according to claim 11, wherein the second air chamber (302) has an opening (3021), wherein along a thickness direction of the circuit board, and the mating hole (202) and the opening are located on opposite sides of the air chamber (300) respectively;
the second detection casing (2112) has an opening part, and the opening part comprises the opening, the circuit board covers the opening;
along a thickness direction of the circuit board, a projection of the opening part on the circuit board is located within an outer contour of the circuit board; and
along the thickness direction of the circuit board, at least a portion of the circuit board (22) and the mating hole (202) are located on opposite sides of the detection casing (201) respectively.

18. The gas detection device according to claim 11, wherein the second detection casing (2112) is connected to the first detection casing (2111), and the detection casing (201) is formed as a single integral structure.

19. The gas detection device according to claim 11, wherein the shell (10) comprises a first casing part (11), the first casing part (11) comprises a first sub-casing part (1101) and a second sub-casing part (1102), and the first sub-casing part (1101) is provided with the ventilation hole (101); and
the first detection unit (2101) is located within the first sub-casing part (1101), at least a portion of the first sub-casing part (1101) is in contact with the first detection unit (2101), the second detection unit (2102) is located within the second sub-casing part (1102), and at least a portion of the second sub-casing part (1102) is in contact with the second detection unit (2102).

20. The gas detection device according to claim 19, wherein the shell (10) comprises a second casing part (12), the circuit board (22) is located within the second casing part (12), and at least a portion of the circuit board is in contact with the second casing part (12); and
both the first sub-casing part (1101) and the second sub-casing part (1102) are connected to the second casing part (12), along a height direction of the gas detection device, the first sub-casing part (1101) and the second sub-casing part (1102) are located on the same side of the second casing part (12).
